(19)

Europäisches Patentamt

European Patent Office

Office européen des brevets

(11) **EP 4 403 630 A1**

(12) **EUROPEAN PATENT APPLICATION**
published in accordance with Art. 153(4) EPC

(43) Date of publication:
**24.07.2024 Bulletin 2024/30**

(21) Application number: **22869406.3**

(22) Date of filing: **16.09.2022**

(51) International Patent Classification (IPC):
*C12N 9/42* (2006.01)     *C12N 15/56* (2006.01)
*C12N 15/81* (2006.01)     *C12N 1/19* (2006.01)
*C12R 1/84* (2006.01)

(52) Cooperative Patent Classification (CPC):
**A23K 20/189; C12N 9/24; C12N 9/2434;
C12N 15/80; C12N 15/81;** C12R 2001/84;
C12R 2001/885

(86) International application number:
**PCT/CN2022/119328**

(87) International publication number:
**WO 2023/041040 (23.03.2023 Gazette 2023/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **18.09.2021   CN 202111097561**

(71) Applicant: **Qingdao Vland Biotech Group Co., Ltd.**
**Qingdao, Shandong 266100 (CN)**

(72) Inventors:
 • **WU, Xiuxiu**
   **Qingdao, Shandong 266100 (CN)**
 • **LI, Rui**
   **Qingdao, Shandong 266100 (CN)**
 • **LI, Yuqiang**
   **Qingdao, Shandong 266100 (CN)**

 • **HUANG, Yijun**
   **Qingdao, Shandong 266100 (CN)**
 • **LI, Xinpei**
   **Qingdao, Shandong 266100 (CN)**
 • **XU, Lihong**
   **Qingdao, Shandong 266100 (CN)**
 • **SONG, Qingqing**
   **Qingdao, Shandong 266100 (CN)**
 • **XIN, Wen**
   **Qingdao, Shandong 266100 (CN)**

(74) Representative: **Boult Wade Tennant LLP**
**Salisbury Square House**
**8 Salisbury Square**
**London EC4Y 8AP (GB)**

Remarks:
The complete document including Reference
Table(s) and the Sequence Listing(s) can be
downloaded from the EPO website

(54)  **HIGH TEMPERATURE RESISTANT MANNANASE MUTANT**

(57)    A high temperature resistant mannanase mutant, comprising at least one mutation site among D49C/I/N, G58D/E/S, H90L, T99Y, K120Y, N124P, T130D/N/P/Q/R/S, Y132F, S136M, S140L/K, T148K, F206Y, A212N/S/P, L257F, S263R, D268A/E/G/P, G273P, S302M/E, S306M/Q, T315P, T353L/F, D360E/N/K, and A362R. The high temperature resistant mannanase mutant has heat resistance significantly higher than the wild type mannanase, and can be widely used in the field of feeds.

EP 4 403 630 A1

## Description

This application claims the priority of Chinese Patent Application No. 202111097561.4, filed with the China National Intellectual Property Administration on September 18, 2021 titled with "High Temperature Resistant Mannanase Mutant".

## FIELD

The present invention relates to the field of genetic engineering and protein modification technology, and particularly relates to a mannanase mutant with high-temperature resistance.

## BACKGROUND

Mannan is a natural polymer with high molecular weight, non-toxic, odorless and high viscosity, which is widely used in the fields of pharmaceuticals, foods and cosmetics, etc. However, mannan existing in cell wall or seed of plants is easy to swell when exposing to water, leading to the great increase in viscosity, which will have an adverse impact on the development and utilization of mannan. For example, in the process of animal feeding, mannan in feed can combine with a large amount of water to form a gelatinous substance with high viscosity, which affects the hydrolysis of feed by digestive enzymes, thus reducing the digestion and utilization efficiency of feed by animals. Therefore, mannan has become a common anti-nutritional factor in the field of feeds.

$\beta$-mannanase (EC3.2.1.78) is a glycoside hydrolase that can degrade $\beta$-1,4-mannosidic bond, and its substrates are linear mannan, glucomannan, galactomannan, galactoglucomannan and the like. Various mannans could be hydrolyzed by $\beta$-mannanase and generated mannose and mannan oligosaccharides (MOS, usually consisting of 2-10 monosaccharide residues).

The research, application and development of $\beta$-mannanase might be traced back to 1950s. In 1958, Courtios et al. first discovered a $\beta$-mannanase from Alfalfa. Williams and Doetsch reported a $\beta$-mannanase obtained from anaerobic rumen Streptococci in 1960. Subsequently, Reese and Shibata et al. first systematically proposed the concept of $\beta$-mannanase in 1965.

$\beta$-mannanases are widely found in plants, animals and microorganisms. For example, $\beta$-mannanase is found in intestinal secretions of some lower class animals and germinant seeds of some plants. At present, most of the reported $\beta$-mannanases are derived from microorganisms, mainly including bacteria, fungi and actinomycete. For the $\beta$-mannanases produced by bacteria, the optimal reaction pH is 5.5-7.0, isoelectric point is 4.0-5.0, and the optimal reaction temperature is 50-70 °C. For the $\beta$-mannanase from fungi, the isoelectric point is usually 4.0-5.0, the optimum reaction temperature is 55-75 °C, and the optimum reaction pH is 4.5-5.5, which belongs to $\beta$-mannanase.

The production of $\beta$-mannanase by microorganisms has the advantages of not being limited by seasons, high expression level, relatively low production cost and to be easily produced by industrial fermentation, so it has become the main way of industrial production of $\beta$-mannanase. However, at present, there is a short-time high temperature stage of 80-90 °C during the production of pellet feed, and the enzyme activity will be greatly lost if $\beta$-mannanase, which is not resistant to high temperature, is directly added to animal feed for granulation. Therefore, improving the thermal stability of $\beta$-mannanase is of great practical significance in the field of feeds.

## SUMMARY

The present invention aims to provide a mannanase mutant. Compared with the wild type mannanase, the heat resistance of the mutants is significantly improved, which is beneficial to its wide application in the field of feeds.

In order to achieve the above-mentioned purpose, the present invention provides the following technical solutions.

The present invention provides a mannanase mutant, which comprises an amino acid sequence having at least 90% identity with SEQ ID NO: 1, and compared with SEQ ID NO: 1, the mannanase mutant comprises at least one amino acid substitution at a position selected from the group consisting of 49, 58, 90, 99, 120, 124, 130, 132, 136, 140, 148, 154, 206, 212, 257, 263, 268, 273, 296, 297, 302, 306, 315, 339, 353, 360 and 362 of SEQ ID NO: 1.

In some embodiments of the present invention, the mutant comprises an amino acid sequence having at least 91%, 92%, 93%, 94%, 95%, 96%, 97%, 98%, or at least 99% identity with SEQ ID NO: 1.

In some more particular embodiments, the mutant comprises an amino acid sequence having at least 99.1%, 99.2%, 99.3%, 99.4%, 99.5%, 99.6%, 99.7%, 99.8%, or at least 99.9% identity with SEQ ID NO: 1.

In some embodiments of the present invention, the mannanase mutant comprises at least one amino acid substitution selected from the group consisting of: D49C/I/N, G58D/E/S, H90L, T99Y, K120Y, N124P, T130D/N/P/Q/R/S, Y132F, S136M, S140L/K, T148K, D154P, F206Y, A212N/S/P, L257F, S263R, D268A/E/G/P, G273P, V296I, T297Y, S302M/E, S306M/Q, T315P, N339F, T353L/F, D360E/N/K and A362R

In some embodiments of the present invention, the mannanase mutant comprises an amino acid substitution or a combination thereof selected from the group consisting of: D49C, D49I, D49N, G58D, G58E, G58S, H90L, T99Y, K120Y, N124P, T130D, T130N, T130P, T130Q, T130R, T130S, Y132F, S136M, S140L, S140K, T148K, D154P, F206Y, A212N, A212S, A212P, L257F, S263R, D268A, D268E, D268G, D268P, G273P, V296I, T297Y, S302M, S302E, S306M, S306Q, T315P, N339F, T353L, T353F, D360E, D360N, D360K, A362R,

D49N/G58D, D49N/H90L, D49N/T99Y, D49N/K120Y, D49N/N124P, D49N/T130R, D49N/T130S, D49N/T130P, D49N/Y132F, D49N/S136M, D49N/S140L, D49N/S140K, D49N/T148K, D49N/D154P, D49N/F206Y, D49N/A212N, D49N/A212S, D49N/A212P, D49C/F206Y, D49I/F206Y, D49N/L257F, D49N/D268A, D49N/D268E, D49N/D268G, D49N/D268P, D49N/G273P, D49N/S302M, D49N/S302E, D49N/S306M, D49N/S306Q, D49N/T315P, D49N/T353L, D49N/D360E, D49C/D360E, D49I/D360E, D49I/D360E, D49N/D360N, D49N/D360K, D49N/A362R, G58D/H90L, G58D/K120Y, G58D/Y132F, G58D/T99Y, G58D/N124P, G58D/T130P, G58D/S136M, G58D/S140K, G58D/T148K, G58D/D154P, G58D/F206Y, G58D/L257F, G58D/D268P, G58D/G273P, G58D/S302M, G58D/S302E, G58D/S306M, G58D/S306Q, G58D/T315P, G58D/T353L, G58D/D360E, H90L/A362R, H90L/G273P, H90L/S302M, H90L/S302E, H90L/S306M, H90L/S306Q, T99Y/N124P, T99Y/T130P, T99Y/S136M, T99Y/S140K, T99Y/T148K, T99Y/D154P, T99Y/F206Y, T99Y/L257F, T99Y/D268P, T99Y/G273P, T99Y/S302M, T99Y/S302E, T99Y/S306M, T99Y/S306Q, T99Y/T315P, T99Y/T353L, T99Y/D360E, K120Y/N124P, K120Y/T130P, K120Y/S136M, K120Y/S140K, K120Y/L257F, K120Y/D268P, K120Y/T315P, K120Y/T353L, K120Y/D360E, K120Y/A362R, N124P/T130P, N124P/S136M, N124P/S140K, N124P/L257F, N124P/D268P, N124P/T315P, N124P/T353L, N124P/D360E, T130P/S136M, T130P/S140K, T130P/L257F, T130P/D268P, T130P/T315P, T130P/T353L, T130P/D360E, S136M/S140K, S136M/L257F, S136M/D268P, S136M/T315P, S136M/T353L, S136M/D360E, S140K/L257F, S140K/D268P, S140K/T315P, S140K/T353L, S140K/D360E, T148K/D154P, T148K/F206Y, T148K/L257F, T148K/D268P, T148K/G273P, T148K/S302M, T148K/S302E, T148K/S306M, T148K/S306Q, T148K/T315P, T148K/T353L, T148K/D360E, D154P/L257F, D154P/D268P, D154P/G273P, D154P/S302M, D154P/S302E, D154P/S306M, D154P/S306Q, D154P/T315P, D154P/T353L, D154P/D360E, F206Y/G273P, F206Y/S302M, F206Y/S302E, F206Y/S306M, F206Y/S306Q, F206Y/T315P, F206Y/T353L, F206Y/D360E, A212N/D268P, A212N/T315P, A212N/T353L, A212N/D360E, L257F/D268P, L257F/T315P, L257F/T353L, L257F/D360E, D268P/T315P, D268P/T353L, D268P/D360E, G273P/S302M, G273P/S302E, G273P/S306M, G273P/S306Q, G273P/T315P, G273P/T353L, G273P/D360E, G273P/A362R, S302M/S306M, S302M/S306Q, S302M/T315P, S302M/T353L, S302M/D360E, S302M/A362R, S302E/S306M, S302E/S306Q, S302E/T315P, S302E/T353L, S302E/D360E, S302E/A362R, S306M/T315P, S306M/T353L, S306M/D360E, S306M/A362R, S306Q/T315P, S306Q/T353L, S306Q/D360E, S306Q/A362R, T315P/T353L, T315P/D360E, T353L/D360E, T315P/A362R, T353L/A362R, D360E/A362R,

D49N/G58D/N124P, D49N/G58D/T130P, D49N/T99Y/N124P, D49N/T99Y/S136M, D268P/T315P/D360E, T130P/L257F/D268P, N124P/T130P/D268P, N124P/T130P/D360E, N124P/S302M/S306M, D49N/G58D/L257F, D49N/G58D/D268P, D49N/G58D/A362R, D49N/T99Y/S140K, D49N/T99Y/L257F, D49N/T99Y/D268P, D49N/G58D/T315P, D49N/G58D/D360E, G58D/N124P/T130P, G58D/N124P/L257F, G58D/N124P/D268P, G58D/D268P/T315P, L257F/D268P/T315P, N124P/T130P/D360K, D49N/T99Y/T315P, D49N/T99Y/T353L, D49N/T99Y/D360E, K120Y/N124P/S140K, K120Y/T130P/L257F, K120Y/S136M/D268P, K120Y/S140K/T353L, N124P/T130P/L257F, N124P/T130P/D268P, A212P/L257F/D268P, D49N/G58D/N124P/T130P, D49N/G58S/T130P/D268P, G58S/N124P/T130P/D268P, G58D/N124P/T130P/D268P, N124P/T130P/L257F/D268P, N124P/T130P/S136M/T353L, N124P/T130P/S136M/D360E, S136M/S140K/T315P/T353L, S136m/L257F/T315P/T353L, S136M/D268P/S306M/T315P, S136M/T315P/F353L/D360E, G58D/T99Y/S140K/T315P/T353L, G58D/N124P/T315P/T353L/D360E, G58S/T99Y/N124P/T130P/D268P, G58D/T99Y/N124P/T130P/D268P, G58D/T130P/T315P/T353L/D360E, G58D/K120Y/N124P/S140K/S136M, D49N/T99Y /S136M/G273P/S302M, T99Y/N124P/T130P/L257F/D268P, D49N/G58D/T99Y/N124P/T130P, D49N/G58D/N124P/T130P/D268P, D49N/G58D/N124P/T130P/T315P, Y132F/L257F/S263R/D268P/T315P, Y132F/L257F/S263R/D268P/T315P, Y132F/L257F/S263R/S306M/T315P, Y132F/G273P/S263R/S306M/T315P, Y132F/G273P/S263R/S306M/D360N, D49N/G58D/N124P/T130P/T353L, D49N/G58D/N124P/T130P/D360E, D49N/G58S/N124P/T130P/S 136M, A212P/L257F/S263R/D268P/T315P, A212P/L257F/S263R/D268P/T315P, A212P/L257F/S263R/S306M/T315P, A212P/G273P/S263R/S306M/T315P, A212P/G273P/S263R/S306M/D360N, D49N/T99Y/S140K/T353L/D360E, D49N/T99Y/L257F/S302M/A362R,

D49N/G58D/N124P/T130P/S140K, D49N/G58D/N124P/T130P/L257F,
G5 8D/N124 P/T13 0P/L25 7F/D26 8P,
D49N/G58D/N124P/T130P/L257F/D268P, G58D/T99Y/N124P/T130P/L257F/D268P,
G58D/N124P/T13OP/S136M/L257F/D268P, G58D/N124P/T130P/S140K/L257F/D268P,
D154P/L257F/D268P/T315P/T353L/D360E, D154P/D268P/S306Q/T315P/T353L/D360E,
D154P/F206Y/D268P/S306Q/T353L/D360E, D49N/N124P/T130P/L257F/T315P/D360E,
D49N/ N124P/T130P/L257F/D268P/D360E, T148K/D154P/D268P/S306Q/T315P/T353L,
T148K/D154P/D268P/S306M/F315P/T353L, D49N/ N124P/T130P/ L257F/D268P/T315P,
D49N/ N124P/F206Y/L257F/D268P/D360K, D49N/ N124P/F206Y/L257F/D268P/T315P,
N124P/T130P/L257F/D268P/T315P/D360E, G58D/N124P/T130P/L257F/D268P/T315P,
G58D/N124P/T130P/L257F/D268P/T353L, G58D/N124P/T130P/L257F/D268P/D360E,
G58D/N124P/T130P/L257F/D268P/T315P, T148K/D154P/ A212P/S306Q/T315P/T353L,
D49N/T130P/L257F/D268P/T315P/D360E, D49N/ N124P/ L257F/D268P/T315P/D360E,
T148K/D154P/ S263R/S306M/T315P/T353L, T148K/D154P/ G273P/S306Q/T315P/T353L,
D49N/ N124P/T130P/ D268P/T315P/D360E,
G58D/N124P/T130P/L257F/D268P/G273P/D360E,
D49N/G5SD/T99Y/N124P/T130P/L257F/D268P,
D49N/G58D/N124P/T130P/S136M/L257F/D268P,
D49N/G58D/N124P/T130P/S140K/L257F/D268P,
D49N/G58D/N124P/T130P/L257F/D268P/T315P,
D49N/G58D/N124P/T130P/L257F/D268P/T353L,
D49N/G58D/N124P/T130P/D268P/T315P/D360E,
D49N/G58D/N124P/T130P/L257F/T315P/D360E,
H90L/N124P T130P/L257F/D268P/G273P/D360E,
H90L/N124P/T13 0P/L2 5 7F/D26 8P/G273P/A3 62R,
D49N/G58D/N124P/T130P/L257F/D268P/D360K,
G58D/N124P/T130P/L257F/D268P/T315P/D360E,
D49N/ N124P/T130P/ L257F/D268P/T315P/D360E,
D49N/G58D/ T130P/ L257F/D268P/T315P/D360E,
D49N/G58D/N124P/ L257F/D268P/T315P/D360E,
D49N/G58D/N124P/T130P/ L257F/D268P/ D360E,
G58D/T99Y/N124P/T130P/L257F/D268P/S302M/S306M,
G58D/N124P/T130P/S136M/L257F/D268P/S302M/T315P,
G58D/N124P/T130P/S140K/L257F/D268P/S302M/D360E,
D49N/G58D/N124P/T130P/L257F/D268P/T315P/T353L,
D49N/G58D/N124P/T130P/S140K/L257F/D268P/T31 SP,
D49N/G58D/N124P/T130P/S136M/L257F/D268P/T315P,
D49N/G58D/T99Y/N124P/T130P/L257F/D268P/F315P,
T148K/S 140K/D 154P/L257F/D268P/T31 SP/T3 53L/D360E,
D49N/G58D/N124P/T130P/L257F/D268P/T315P/D360E,
D154P/D268P/S302M/S306M/T315P/T353L/D360E/A362R,
D 154P/D268P/S3 02M/S3 06M/T31 SP/T3 53 L/D3 60E/A3 62R,
D49N/G58D/N124P/T130P/S140K/L257F/D268P/T353L,
D49N/G58D/N124P/T130P/S136M/L257F/D268P/T353L,
D49N/G58D/H90L/T130P/S136M/L257F/S306M/T353L,
D49N/G5 8D/H90L/T13 0P/S13 6M/G273P/S3 06M/T3 53L,
D49N/G58D/N124P/T130P/S136M/S140K/L257F/D268P,
D49N/G5 8D/N124P/T130P/L257F/245P/G273P/A362R,
H90L/T99Y/N124P/T130P/L257F/D268P/S302M/S306M,
H90L/N124P/T130P/S136M/L257F/D268P/S302M/T315P,
H90L/N124P/T130P/S140K/L257F/D268P/S302M/D360E,
T99Y/N124P/T130P/Y132F/D268P/S302P/S306M/T315P,
D49N/G58D/N124P/F130P/L257F/D268P/T315P/D360E/T353L,
D49N/G58D/N124P/T130P/S136M/L257F/D268P/T315P/D360E,
D49N/G58D/N124P/T130P/S140K/L257F/D268P/T315P/D360E,
D49N/G58D/N124P/T130P/L257F/D268P/T315P/T353L/D360E,
D49N/G58D/T99Y/N124P/T130P/L257F/D268P/T315P/D360E,
T99Y/N124P/T130P/L257F/D268P/S302M/T315P/D360E/A362R,

K120Y/N124P/T130P/S136M/L257F/D268P/T315P/T3 53L/D360E,
D49N/G58D/N124P/T130P/S136M/S140K/L257F/D268P/T315P/T3 53L/D360E,
D49N/G58D/N124P/T130P/S136M/L257F/D268P/V296I/T297Y/T315P/D360E,
D154P/L257F/D268P/S302M/S306M/T315P/T353L/D360E/A362R,
D49N/G58D/N124P/T130P/S136M/S140K/L257F/D268P/T315P/D360E,
D154P/L257F/D268P/S302M/S306M/T315P/T353L/D360E/A362R,
D49N/G58D/N124P/T130P/S136M/L257F/D268P/T31SP/T353L/D360E,
D49N/G58D/N124P/T130P/S140K/L257F/D268P/T315P/T353L/D360E,
D49N/G58D/N124P/F130P/S136M/L257F/D268P/V296I/F315P/D360E,
D49N/G58D/N124P/T130P/S136M/L257F/D268P/T297Y/T315P/D360E,
D49N/G58D/N124P/T130P/L257F/D268P/G273P/S306M/T315P/T353L/D360E,
D49N/G58D/T99Y/N124P/T130P/L257F/D268P/S302M/T315P/D360E/A362R,
G58D/T99Y/N124P/T130P/S136M/S140K/D154P/L257F/D268P/T315P/D360E/A362R,
T130P/Y132F/S136M/S140K/D268P/G273P/S302M/S306M/T315P/T353L/D360E/A362R,
D49N/G58D/T99Y/N124P/T130P/S136M/S140K/L257F/D268P/T315P/T353L/D360K,
D49N/G58D/H90L/K120Y/N124P/T130P/S136M/L257F/D268P/T31SP/T353L/D360E,
D49N/G58D/T99Y/N124P/T130P/S136M/S140K/L257F/D268P/T315P/T353L/D360E,
D49N/G58D/N124P/T130P/T148K/S140K/D154P/L257F/D268P/T315P/T353L/D360E,
D49N/G58D/N124P/T130P/S136M/L257F/D268P/V296I/T297Y/T315P/N339F/D360E,
D49N/G58D/T99Y/N124P/T1130P/Y132F/D154P/D268P/G273P/S302P/S306M/T315P,
T99Y/N124P/T130P/Y132F/S136M/S140K/D268P/S302M/S306M/T315P/T353L/D360E/ A362R,
H90L/N124P/T130P/Y132F/S136M/S140K/D268P/S302M/S306M/T315P/T353L/D360E/ A362R,
H90L/K120Y/T130P/Y132F/S136M/S140K/D268P/S302M/S306M/T315P/T353L/D360K/ A362R,
N124P/T13 0P/Y132F/S13 6M/S140K/L25 7F/D268P/S3 02M/S3 06M/T315P/F3 5 3L/D360E/ A362R,
T99Y/S113M/S117K/N124P/T130P/Y132F/D268P/S302P/S306M/T315P/T330L/D337E/A 339R,
T99Y/N124P/T130P/Y132F/S136M/S140K/L257F/D268P/S302M/S306M/T315P/T353L/D 360E/A362R,
T148K/D154P/F206Y/A212P/L257F/S263R/D268P/G273P/S302M/S306M/T315P/T353F/ D360E/A362R,
T99Y/T148K/D154P/F206Y/A212P/L257F/S263R/D268P/G273P/S302M/S306M/T315P/T 353F/D360E/A362R,
T99Y//N124P/T148K/D154P/F206Y/A212P/L257F/S263R/D268P/G273P/S302M/S306M/
T315P/T353F/D360E/A362R,
D49N/G58D/N124P/T130P/T148K/A212P/L257F/S263R/D268P/G273P/S302M/S306M/T
315P/T353F/D360E/A362R,
D49N/G58D/T99Y/T130P/T148K/A212P/L257F/S263R/D268P/G273P/S302M/S306M/T3
15P/T353F/D360E/A362R,
D49N/G58D/T99Y/T130P/T148K/A212P/L257F/S263R/D268P/G273P/S302M/S306M/T3
15P/T353F/D360E/A362R,
D49N/G58D/T99Y/T130P/T148K/D154P/A212P/L257F/S263R/D268P/G273P/S302M/S30
6M/T315P/T353F/D360E/A362R,
D49N/G58D/T99Y/T130P/T148K/D154P/A212P/L257F/S263R/D268P/G273P/S302M/S30
6M/T315P/T353F/D360E/A362R,
G58D/T99Y/N124P/T130P/D154P/F206Y/A212P/L257F/S263R/D268P/G273P/S302M/S3
06M/T315P/T353F/D360E/A362R,
G58D/H90L/T99Y/N124P/T130P/D15 4P/F206Y/A212P/L2 5 7F/S263R/D26 8P/G273 P/S 3 0
2M/S306M/T315P/T353F/D360K/A362R,
G58D/H90L/T99Y/N124P/T130P/D154P/F206Y/A212P/L257F/S263R/D268P/G273P/S30
2E/S306M/T315P/T353F/D360E/A362R,
G58D/H90L/T99Y/N124P/T130P/D154P/F206Y/A212P/L257F/S263R/D268P/G273P/S30
2E/S306M/T315P/T353F/D360N/A362R, and
G58D/H90L/T99Y/N124P/T130P/D154P/F206Y/A212P/L257F/S263R/D268P/G273P/S30
2E/S306M/T315P/T353L/D360K/A362R.

The present invention further provides a DNA molecule encoding the mannanase mutant.

The present invention further provides a recombinant expression plasmid comprising the above DNA molecule.

The present invention further provides a host cell comprising the above recombinant expression plasmid.

The heat resistance of the recombinant mannanase mutant is significantly enhanced, wherein the mannanase mutant is produced by transforming the above plasmid into host cells.

In some embodiments of the present invention, the host cell is *Pichia pastoris.*

In some embodiments of the present invention, the host cell is *Trichoderma reesei.*

The present invention further provides the use of the mannanase mutant in the field of feeds.

On the basis of wild-type mannanase M20, the present invention provides a mannanase mutant comprising at least one mutation site selected from the group consisting of: D49C/I/N, G58D/E/S, H90L, T99Y, K120Y, N124P, T130D/N/P/Q/R/S, Y132F, S136M, S140L/K, T148K, D154P, F206Y, A212N/S/P, L257F, S263R, D268A/E/G/P, G273P, S302M/E, S306M/Q, T315P, T353L/F, D360E/N/K and A362R. Compared with wild-type mannanase, the residual enzyme activity of the mutant provided by the present invention generally improved by 69.9%-429.6%, and the heat resistance is significantly improved, after being treated at 80°C for 3 minutes. Among them, the mutant comprising a single mutation site of N124P and the mutant comprising a single mutation site of D360K show the best heat resistance, and the residual enzyme activity is as high as 51.81% and 60.69%, respectively, achieving unexpected technical effects.

In summary, the heat resistance of the mannanase mutants provided by the present invention is significantly improved, which is beneficial to its wide application in the field of feeds.

## DETAILED DESCRIPTION

The present invention discloses a mannanase mutant, a preparation method and a use thereof, a DNA molecule encoding the mannanase mutant, a vector, and a host cell. Those skilled in the art can learn from the content of the present invention and make appropriate improvement on the process parameters to achieve the present invention. The method and the use of the present invention have been described through the preferred embodiments, and it is obvious that the method and use described herein may be changed or appropriately modified and combined to realize and use the technology of the present invention by those skilled in the art.

The present invention employs conventional techniques and methods used in the fields of genetic engineering and molecular biology, such as the methods described in MOLECULAR CLONING: A LABORATORY MANUAL, 3nd Ed. (Sambrook, 2001) and CURRENT PROTOCOLS IN MOLECULAR BIOLOGY (Ausubel, 2003). These general references provide definitions and methods known to those skilled in the art. However, those skilled in the art can adopt other conventional methods, experimental schemes, and reagents in the field on the basis of the technical solutions described in the present invention, and are not limited to the limitations of the particular embodiments of the present invention. For example, the following experimental materials and reagents might be used.

Strains and vectors: *Escherichia. coli* DH5α, *Pichia pastoris* GS115, vector pPIC9k, Amp and G418 were purchased from Invitrogen.

Enzymes and kits: PCR enzymes and ligases were purchased from Takara, restriction enzymes were purchased from Fermentas, lyase was purchased from Sigma, plasmid extraction kits and gel purification recovery kits were purchased from Omega, and GeneMorph II random mutagenesis kits were purchased from Beijing Biomars Biological Technology Co., Ltd.

The formula of medium is as follows:

*Escherichia. coli* culture medium (LB medium): 0.5% yeast extract, 1% peptone, 1% NaCl, pH 7.0;

Yeast culture medium (YPD medium): 1% yeast extract, 2% peptone, 2% glucose;

Yeast screening medium (MD plate): 2% peptone, 2% agarose;

BMGY medium: 2% peptone, 1% yeast extract, 100 mM potassium phosphate buffer (pH 6.0), 1.34% YNB, $4\times10^{-5}$% biotin, 1% glycerol;

BMMY medium: 2% peptone, 1% yeast extract, 100 mM potassium phosphate buffer (pH 6.0), 1.34% YNB, $4\times10^{-5}$% biotin, 0.5% methanol;

LB-AMP medium: 0.5% yeast extract, 1% peptone, 1% NaCl, 100 μg/mL ampicillin, pH 7.0;

LB-AMP plate: 0.5% yeast extract, 1% peptone, 1% NaCl, 1.5% agar, 100 μg/mL ampicillin, pH 7.0;

Upper layer medium: 0.1% $MgSO_4$, 1% $KH_2PO_4$, 0.6% $(NH_4)_2SO_4$, 1% glucose, 18.3% sorbitol, 0.35% agarose;

Lower layer medium (plate): 2% glucose, 0.5% $(NH_4)_2SO_4$, 1.5% $KH_2PO_4$, 0.06% $MgSO_4$, 0.06% $CaCl_2$, 1.5% agar.

The present invention will be further illustrated below in conjunction with examples.

**Example1 Gene cloning and expression plasmid construction of wild-type mannanase**

The genome of *Aspergillus niger* was used as a template for PCR amplification, and PCR primers M20-F1 and M20-R1 were as follows:

M20-F1: GCTGAATTCGGCCTCCAATTCACCATTGATGGCG (the underlined sequence is the recognition site of restriction enzyme EcoRI);

M20-R1: CTGGCGGCCGCTTAGGCGCTATCAATAGCAG (the underlined sequence is the recognition site of restriction enzyme NotI).

PCR products were recovered from gel, ligated with pEASY-T vector, transformed into *E.coli* DH5α, and the correct transformants were selected for sequencing. Sequencing results showed that the nucleotide sequence of the gene fragment obtained by amplification was SEQ ID NO: 2 and the encoded amino acid sequence was SEQ ID NO: 1, which indicated that the expression plasmid was successfully constructed and named pPIC9K-M20. Through NCBI BLAST alignment, it was found that the SEQ ID NO: 1 had 100% sequence identity with the sequence of the acidic mannanase from *Aspergillus Niger.* Thus, it was determined that the gene obtained by PCR was a mannanase gene, named M20.

**Example 2 Screening of high temperature resistant mannanase mutants**

In order to improve the heat resistance of acidic mannanase M20, a large number of mutants of the enzyme were screened by directed evolution technique.

M20 gene was served as the template, and the primers M20-F1 and M20-R1 were used to perform PCR amplification by GeneMorph II Random Mutagenesis Kit (Beijing Biomars), followed by recovering the PCR product from gel. After being digested with EcoRI and NotI, the PCR product was ligated with pET21a vector digested with the same enzymes. The ligation product was transformed into *E. coli* BL21 (DE3) and then the cells were spread on LB+Amp plate for culturing upside down at 37°C. After the transformation, single colonies were picked one by one with toothpicks and transferred to a 96-well plate. Each well on the plate was added with 150 μl of LB+Amp medium containing 0.1 mM IPTG, followed by cultured at 37°C for about 6 hours with shaking at 220 rpm. Afterwards, the culture was centrifuged and the supernatant was discarded. The bacteria cells were resuspended in buffer, and frozen and thawed repeatedly to obtain *E. coli* cell lysate containing mannanase.

30 μL of lysate was taken from each well and transferred to two new 96-well plates, respectively. Then, one of the plates was treated at 75°C for 5 min. Afterwards, 30 μL of substrate was added to each well in both 96-well plates, and reaction was carried out at 37°C for 30 min. The reducing sugar was determined by DNS method, and the enzyme activity levels of different mutants were calculated respectively.

The experimental results showed that different mutants exhibited different activities after high temperature treatment. Some mutations had no effect on the heat resistance of mannanase M20, while some made it even worse. In addition, there were some mutations, which improved the heat resistance of mannanase, but changed its enzymatic properties significantly. These mutants did not meet the desired requirements. Finally, mutation sites that not only significantly improved the heat resistance of mannanase but also did not affect its original enzymatic properties were selected, which were as follows: D49C, D49I, D49N, G58D, G58E, G58S, H90L, T99Y, K120Y, N124P, T130D, T130N, T130P, T130Q, T130R, T130S, Y132F, S136M, S140L, S140K, T148K, D154P, F206Y, A212N, A212S, A212P, L257F, S263R, D268A, D268E, D268G, D268P, G273P, S302M, S302E, S306M, S306Q, T315P, T353L, T353F, D360E, D360N, D360K and A362R.

Based on the wild-type mannanase M20, the present invention provides mannanase mutants which comprises one mutation selected from the group consisting of D49C, D49I, D49N, G58D, G58E, G58S, H90L, T99Y, K120Y, N124P, T130D, T130N, T130P, T130Q, T130R, T130S, Y132F, S136M, S140L, S140K, T148K, D154P, F206Y, A212N, A212S, A212P, L257F, S263R, D268A, D268E, D268G, D268P, G273P, S302M, S302E, S306M, S306Q, T315P, T353L, T353F, D360E, D360N, D360K and A362R.

The present invention further provided a mannanase mutant comprising at least 2, at least 3, at least 4, at least 5, at least 6, at least 7, at least 8, at least 9, at least 10, at least 11, at least 12, at least 13, at least 14, at least 15, at least 16, at least 17, or at least 18 mutations selected from the group consisting of D49C/I/N, G58D/E/S, H90L, T99Y, K120Y, N124P, T130D/N/P/Q/R/S, Y132F, S136M, S140L/K, T148K, D154P, F206Y, A212N/S/P, L257F, S263R, D268A/E/G/P, G273P, V296I, T297Y, S302M/E, S306M/Q, T315P, T353L/F, D360E/N/K and A362R.

Referring to the amino acid sequence of the mutants, the nucleotide sequences encoding the mannanase mutants were obtained.

**Example 3 Expression of mannanase in *Pichia pastoris***

3.1 Construction of expression plasmid

According to the codon bias of *Pichia pastoris,* the sequences of wild-type mannanase M20 and the mutants were optimized. The genes were synthesized by Shanghai Generay Biotechnology Co., Ltd., and two restriction sites of enzymes EcoRI and NotI were added to the 5' and 3' ends of the synthetic sequence.

Following the method described in Example 1, the DNA of the synthesized mutant was digested with EcoRI and NotI, and then ligated with the pPIC-9K vector (digested with the same enzymes) at 16°C overnight. The ligation product was transformed into E. *coli* DH5a and then spread on LB+Amp plate for culturing upside down at 37°C. After the transformants appeared, the positive clones were verified by colony PCR, and the correct expression plasmid of recombinant mutant was obtained after sequencing verification.

3.2 Construction of engineered strains of *Pichia pastoris*

3.2.1 The preparation of competent cell of yeast

*Pichia pastoris* GS115 strain was activated on an YPD plate. After culturing at 30°C for 48 hours, single clone of the activated GS115 was inoculated into 6 mL of YPD liquid medium and cultured at 220 rpm at 30°C for about 12 hours. After that, the cells were transferred into an Erlenmeyer flask containing 30 mL of YPD liquid medium and cultured at 220 rpm at 30°C for about 5 hours. The cell density was detected by an ultraviolet spectrophotometer. After the OD600 value was in the range of 1.1-1.3, 4 mL of cells was collected into a sterilized EP tube by centrifugation at 4°C, 9,000 rpm for 2 min. The supernatant was carefully discarded, and the remaining supernatant was absorbed with sterile filter paper. Subsequently, the cells were resuspended in 1 mL of pre-cooled sterilized water, followed by centrifugation at 4°C, 9,000 rpm for 2 min. The supernatant was carefully discarded, and the cells were washed with 1 mL sterile water, followed by centrifugation at 4°C, 9,000 rpm for 2 min. The supernatant was carefully discarded, and the cells were resuspended in 1 mL of pre-cooled sorbitol (1 mol/L), followed by centrifugation at 4 °C, 9,000 rpm for 2 min. The supernatant was carefully discarded, and the cells were gently resuspended in 100-150 μl of the pre-cooled sorbitol (1 mol/L).

3.2.2 Transformation and screening

The expression plasmids constructed in Example 1 and Example 3.1 were linearized with SacI. After the linearized fragments were purified and recovered, they were transformed into *Pichia pastoris* GS115 by electroporation. The recombinant strains of *Pichia pastoris* were screened on MD plates, and then transformants with multiple copies were screened on an YPD plate containing different concentrations of geneticin (0.5 mg/mL-8 mg/mL).

The obtained transformants were transferred to BMGY medium and cultured with shaking at 30°C and 250 rpm for 1d; then transferred to BMMY medium and cultured with shaking at 250 rpm, 30°C. 0.5% methanol was added every day to induce expression for 4 days. The cells were removed by centrifugation at 9000 rpm for 10 min to obtain supernatants from fermentation containing wild-type mannanase M20 and mutants respectively.

3.3 Method for measuring enzyme activity of mannanase

(1) Definition of enzyme activity unit of mannanase

At 37°C and pH 5.5, the amount of enzyme required to degrade and release 1 μmol reducing sugar per minute from a 3 mg/mL mannan solution is defined as one enzyme-activity unit U.

(2) Determination method of enzyme activity

(2.1) Establishment of standard curve:

4.0 mL of acetic acid-sodium acetate buffer solution was taken and added to 5.0 mL of DNS reagent. The obtained mixture was heated in boiling water bath for 5 min then cooled to room temperature by tap water. The volume was adjusted to 25.0 mL with water to make a standard blank sample.

1.00 ml, 2.00 ml, 3.00 ml, 4.00 ml, 5.00 ml, 6.00 ml and 7.00 mL of mannose solution (pH 5.5) were taken respectively, and the total volume was adjusted to 100 mL with acetic acid-sodium acetate buffer solution respectively, to prepare D-mannose standard solutions with concentrations of 0.10-0.70 mg/ml.

2.00 mL of mannose standard solutions of the above concentration series (in two replicates) were taken and added into calibration test tubes, respectively. And then 2 mL of acetic acid-sodium acetate buffer solution and 5 ml of DNS reagent were added to each tube. The test tubes were subjected to electromagnetic oscillation for 3s, heated in boiling water bath for 5 min and cooled to room temperature by tap water. The volume of each tube was adjusted to 25 mL with water. The standard blank sample was used as control for zero adjustment, and the OD value was measured at 540 nm.

The standard curve was plotted with mannose concentration as y axis and OD value as the x axis. The standard curve should be re-plotted every time when a new batch of DNS reagent was prepared.

(2.2) Determination of enzyme activity:

10.0 mL of mannan solution was taken and equilibrated at 37 °C for 10min.

10.0 mL of properly diluted enzyme solution was taken and equilibrated at 37 °C for 10 min.

2.00 mL of properly diluted enzyme solution (after equilibration at 37 °C) was taken and added to the calibration test tube, and 5 ml of DNS reagent was added. The test tube was subjected to electromagnetic oscillation for 3 s. 2.0 mL of mannan solution was added to the tube, kept at 37 °C for 30 min, heated in boiling water bath for 5 min and cooled to room temperature by tap water. The volume was adjusted to 25 mL, and then subjected to electromagnetic oscillation for 3 s. The standard blank sample was used as the blank control, the absorbance $A_B$ was determined at 540 nm.

2.00 mL of properly diluted enzyme solution (after equilibration at 37 °C) was taken and added to the calibration test tube, and 2.0 ml of mannan solution (after equilibration at 37 °C) was also added. The test tube was subjected to electromagnetic oscillation for 3 s and kept at 37 °C for 30 min. 5.0 mL DNS reagent was added. The test tube was subjected to electromagnetic oscillation for 3 s and enzymatic hydrolysis was carried out. Then, the tube was heated in boiling water bath for 5 min and cooled to room temperature by tap water. The volume was adjusted to 25 mL and subjected to electromagnetic oscillation for 3 s. The standard blank sample was used as the blank control, the absorbance $A_E$ was determined at 540 nm.

The calculation formula of enzyme activity is as follow:

$$X_D = \frac{[(A_E - A_B) \times K + C_0]}{M \times t} \times N \times 1000$$

.

In the formula: $X_D$ is the activity of mannanase in diluted enzyme solution, U/ml; $A_E$ is the absorbance of the enzyme reaction solution; $A_B$ is the absorbance of enzyme blank solution; K is the slope of the standard curve; $C_0$ is the intercept of the standard curve; M is the molar mass of xylose, 180.2 g/mol; t is the reaction time of enzymolysis, min; N is the dilution multiple of enzyme solution; 1000 is the conversion factor, 1 mmol=1000 $\mu$mol.

(3) Results of enzyme activity determination

The enzyme activity was measured according to the above method. The results showed that the enzyme activity of the fermentation supernatant from the recombinant *Pichia pastoris* strain expression mannanase M20 or mutants was 200-500 U/mL.

Example 4 Expression of mannanases in *Trichoderma reesei*

According to the codon bias of *Trichodenna reesei,* the sequences of wild-type mannanase M20 and the mutants were optimized. The genes were synthesized by Shanghai Generay Biotechnology Co., Ltd., and two restriction sites of enzymes KpnI and MluI were added to the 5' and 3' ends of the synthetic sequence.

4.1 Construction of expression vector

The synthesized mannanase gene fragment and pSCIG vector were digested with restriction enzymes KpnI and MluI, respectively. The digested products were purified by gel purification kit and ligated together using T4 DNA ligase. The ligation products were transformed into *E.coli* DH5$\alpha$. The clones were selected by ampicillin and verified by sequencing. After sequencing verification, the recombinant plasmid comprising mannanase gene was obtained.

4.2 Construction of recombinant strain of *Trichoderma reesei*

(1) The preparation of protoplast

The spore suspension from *Trichodenna reesei* UE host cells was inoculated on a PDA plate, and cultured at 30 °C for 6 days. After the spores were abundant, a colony of about 1 cm × 1 cm was cut and transferred into liquid culture medium containing 120 mL of YEG+U (0.5% yeast powder, 1% glucose, and 0.1% uridine), and cultured with shaking at 30 °C and 220 rpm for 14-16 h.

The mycelia were collected by filtration with sterile gauze, and washed once with sterile water. The mycelia were transferred into an Erlenmeyer flask containing 20 mL of 10 mg/mL lysing enzyme solution (Sigma L1412) and cultured at 30°C, 90 rpm for 1-2 h. The process of protoplast transformation was observed and detected with a microscope.

20 mL of pre-cooled 1.2 M sorbitol (1.2 M sorbitol, 50 mM Tris-Cl, 50 mM CaCl$_2$) was added to the above flask and shaken well gently. The filtrate was collected by using the sterile Miracloth filtration material and centrifuged at 3000 rpm at 4°C for 10 min. The supernatant was discarded, and 5 mL of pre-cooled sorbitol solution (1.2M) was added to resuspend the cells. The mixture was centrifuged at 3000 rpm at 4°C for 10 min, the supernatant was discarded, and appropriate amount of the pre-cooled sorbitol (1.2M) was added to resuspend the cells and the cells were aliquoted (200 μL/ tube, with a concentration of 10$^8$ protoplast /mL). (2) Transformation of expression vector

The following operations were all performed on ice. 10 μg of the recombinant plasmids constructed above was added to 7 mL sterile centrifuge tube containing 200 μL of protoplast solution, and then 50 μL of 25% PEG (25% PEG, 50 mM Tris-Cl, 50 mM CaCl$_2$) was added. The mixture was mixed well by flicking the bottom of the tube, and placed on ice for 20 minutes. 2 mL of 25% PEG was added, mixed well and allowed to stand for 5 minutes at room temperature. 4 mL of 1.2 M sorbitol was added and mixed gently. The mixture was added to the upper medium that had been melted and kept at 55°C. After being mixed gently, the mixture was spread on the pre-prepared lower medium plate and cultured at 30°C for 5-7 days until transformants appeared. The transformants were picked and transferred to the lower medium plate for re-screening, and the colonies with smooth edges were the positive transformants.

According to the above method, the recombinant *Trichodenna reesei* strains expressing wild-type mannanase M20 or mutants were respectively constructed.

(3) Fermentation verification and enzyme activity determination

The engineered *Trichoderma reesei* strains constructed above were inoculated on the PDA solid plate respectively, and cultured upside down in a constant temperature incubator at 30°C for 6-7 days. After the spores were abundant, two colonies with a diameter of 1 cm were taken and inoculated into 250 mL Erlenmeyer flask containing 50 mL fermentation medium (1.5% glucose, 1.7% lactose, 2.5% corn steep liquor, 0.44%(NH$_4$)$_2$SO$_4$, 0.09% MgSO$_4$, 2% KH$_2$PO$_4$, 0.04% CaCl$_2$, 0.018% Tween-80, 0.018% trace element), cultured at 30°C for 48 hours and then cultured at 25°C for 48 hours. The fermentation solutions were centrifuged to obtain fermentation supernatants containing wild-type mannanase M20 and mutants respectively.

According to the above method, the enzyme activity was detected, and the results showed that the enzyme activity of the fermentation supernatants from recombinant *Trichodenna reesei* strains expressing wild-type mannanase M20 or mutant was 230-510 U/mL.

**Example 5 Identification and analysis of enzymatic properties of mannanase**

1. Analysis of optimal pH

Disodium hydrogen phosphate-citric acid buffers with pH values of 2.0, 2.5, 3.0, 4.0, 4.5, 5.0, 5.5, 6.0, 7.0 and 8.0 were respectively used to dilute the supernatants after fermentation of recombinant *Pichia pastoris* strains constructed above, and mannan substrate was also prepared using buffers with corresponding pH values, respectively. The enzyme activity of mannase was measured at 37°C and the relative enzyme activity was calculated by taking the highest enzyme activity as 100%.

The results showed that the optimal pH value of both wild-type mannanase M20 and mutants was 4.0, indicating that the mutations did not change the optimal pH value of mannanase M20.

2. Analysis of optimal temperature

The enzyme activity of mannanase in the supernatants after fermentation of the recombinant *Pichia pastoris* strains constructed above was determined at various temperatures of 30°C, 35°C, 40°C, 45°C, 50°C, 55°C, 60°C, 65°C, 70°C, 75°C, 80°C, 85°C, 90°C, under pH of 5.5. The relative enzyme activity was calculated by taking the highest enzyme activity as 100%.

The results showed that the optimal reaction temperature of wild-type mannanase M20 was 70°C. The optimal reaction temperature of mannanase mutants was 72-77°C.

3. Analysis of heat resistance

The fermentation supernatants of recombinant *Pichia pastoris* strains were diluted to about 20 U/mL with acetic acid-sodium acetate buffer with pH5.5, and treated at 80°C for 3 min. The enzyme activity of mannanase was determined, and the residual enzyme activity was calculated by taking enzyme activity of the untreated sample as 100%. The results are shown in Table 1.

Residual enzyme activity (%) = enzyme activity of treated samples / enzyme activity of untreated samples × 100%.

Table 1. Comparison of the heat resistance of mannanase mutants

| Mannanase Mutants with Single Mutation | Residual Enzyme Activity |
|---|---|
| wild-type M20 | 11.46% |
| D49I | 20.34% |
| D49C | 21.92% |
| D49N | 31.74% |
| G58D | 27.79% |
| G58E | 23.22% |
| G58S | 23.82% |
| H90L | 19.47% |
| T99Y | 21.93% |
| K120Y | 32.83% |
| N124P | 51.81% |
| T130D | 26.14% |
| T130N | 23.51% |
| T130Q | 21.87% |
| T130R | 19.97% |
| T130P | 37.31% |
| T130S | 22.13% |
| Y132F | 23.49% |
| S136M | 26.74% |
| S140L | 24.79% |
| S140K | 19.48% |
| T148K | 29.37% |
| D154P | 25.93% |
| F206Y | 28.67% |
| A212N | 23.06% |
| A212S | 24.01% |
| A212P | 21.46% |
| L257F | 37.52% |

(continued)

| Mannanase Mutants with Single Mutation | Residual Enzyme Activity |
| --- | --- |
| S263R | 43.75% |
| D268A | 23.88% |
| D268G | 19.79% |
| D268E | 20.07% |
| D268P | 28.73% |
| G273P | 25.35% |
| S302M | 20.20% |
| S302E | 40.78% |
| S306M | 39.51% |
| S306Q | 25.98% |
| T315P | 20.03% |
| T353L | 22.47% |
| T353F | 33.48% |
| D360E | 29.47% |
| D360N | 39.01% |
| D360K | 60.69% |
| A362R | 35.01% |

As can be seen from the results shown in Table 1, compared with wild-type mannanase M20, the residual enzyme activities of the mutants with single mutation were increased by 69.9%-429.6%, after being treated at 80°C for 3 min, indicating that the heat resistance of these mutants was significantly improved. Among them, N124P mutant and D360K mutant have the best heat resistance. After being treated at 80°C for 3 minutes, the residual enzyme activities was as high as 51.81% and 60.69% of the untreated enzyme, respectively, showing unexpected technical effect.

The high temperature resistant mannanase provided by the present invention is suitable to be widely used in the field of feeds.

The high temperature-resistant mannanase provided by the present invention has been introduced in detail above. The principle and implementation of the present invention are illustrated by using specific embodiments herein. The above descriptions of the embodiments are only used to facilitate understanding of the method and the core idea of the present invention.

# EP 4 403 630 A1

## Sequence Listing

```
<?xml version="1.0" encoding="UTF-8"?>
<!DOCTYPE ST26SequenceListing SYSTEM "ST26SequenceListing_V1_3.dtd" PUBLIC
"-//WIPO//DTD Sequence Listing 1.3//EN">
<ST26SequenceListing productionDate="2022-09-15" softwareVersion="2.1.1"
softwareName="WIPO Sequence" fileName="OP221595. Sequence Listing.xml"
dtdVersion="V1_3"><ApplicationIdentification><IPOfficeCode>CN</IPOfficeCode><A
pplicationNumberText/><FilingDate/></ApplicationIdentification><ApplicantFileR
eference>OP221595</ApplicantFileReference><EarliestPriorityApplicationIdentifi
cation><IPOfficeCode>CN</IPOfficeCode><ApplicationNumberText>202111097561.4</A
pplicationNumberText><FilingDate>2021-09-18</FilingDate></EarliestPriorityAppl
icationIdentification><ApplicantName languageCode="zh">Qingdao Weilan Biology
Group CO., LTD </ApplicantName><ApplicantNameLatin>Qingdao Weilan Biology Group
Co. , Ltd.</ApplicantNameLatin><InventionTitle languageCode="zh"> high
temperature-resistant mannanase
</InventionTitle><SequenceTotalQuantity>2</SequenceTotalQuantity><SequenceData
sequenceIDNumber="1"><INSDSeq><INSDSeq_length>362</INSDSeq_length><INSDSeq_mol
type>AA</INSDSeq_moltype><INSDSeq_division>PAT</INSDSeq_division><INSDSeq_feat
ure-table><INSDFeature><INSDFeature_key>source</INSDFeature_key><INSDFeature_l
ocation>1..362</INSDFeature_location><INSDFeature_quals><INSDQualifier><INSDQu
alifier_name>mol_type</INSDQualifier_name><INSDQualifier_value>protein</INSDQu
alifier_value></INSDQualifier><INSDQualifier
id="q2"><INSDQualifier_name>organism</INSDQualifier_name><INSDQualifier_value>
synthetic
construct</INSDQualifier_value></INSDQualifier></INSDFeature_quals></INSDFeatu
re></INSDSeq_feature-table><INSDSeq_sequence>LPKASPAPSTSSSAASTSFASTSGLQFTIDGET
GYFAGTNSYWIGFLTDNADVDLVMGHLKSSGLKILRVWGFNDVTSQPSSGTVWYQLHQDGKSTINTGADGLQRLDYVV
SSAEQHDIKLIINFVNYWTDYGGMSAYVSAYGGSGETDFYTSDTMQSAYQTYIKTVVERYSNSSAVFAWELANEPRCP
SCDTSVLYNWIEKTSKFIKGLDADRMVCIGDEGFGLNIDSDGSYPYQFSEGLNFTMNLGIDTIDFGTLHLYPDSWGTS
DDWGNGWITAHGAACKAAGKPCLLEEYGVTSNHCSVEGSWQKTALSTTGVGADLFWQYGDDLSTGKSPDDGNTIYYGT
SDYQCLVTDHVAAIDSA</INSDSeq_sequence></INSDSeq></SequenceData><SequenceData
sequenceIDNumber="2"><INSDSeq><INSDSeq_length>1089</INSDSeq_length><INSDSeq_mo
ltype>DNA</INSDSeq_moltype><INSDSeq_division>PAT</INSDSeq_division><INSDSeq_fe
ature-table><INSDFeature><INSDFeature_key>source</INSDFeature_key><INSDFeature
_location>1..1089</INSDFeature_location><INSDFeature_quals><INSDQualifier><INS
DQualifier_name>mol_type</INSDQualifier_name><INSDQualifier_value>other
DNA</INSDQualifier_value></INSDQualifier><INSDQualifier
id="q4"><INSDQualifier_name>organism</INSDQualifier_name><INSDQualifier_value>
synthetic
```

13

construct</INSDQualifier_value></INSDQualifier></INSDFeature_quals></INSDFeature></INSDSeq_feature-table><INSDSeq_sequence>ctgccgaaagcctcccctgcaccgagcaccagc agcagtgctgcctccacctccttcgccagcacctccggcctccaattcaccattgatggcgaaactggctacttcgcc ggaacgaacagctactggatcggtttcctcactgacaacgcggacgtcgacctcgtcatgggccacctgaagtcgtcc ggcctcaagatcctccgcgtgtggggcttcaacgatgtcacctcgcagccctcctccggcacagtctggtaccaactg caccaggacggcaaatcgacaatcaacacgggtgccgacggtctccagcgcctcgactacgtcgtctcgtctgccgaa cagcacgacatcaaactcatcatcaacttcgtcaactactggaccgattacggtggtatgtctgcgtacgtgagcgcg tatggcggatccggcgagacggatttctataccagtgataccatgcagagtgcctatcagacatatatcaagacggtc gtggagcggtacagtaactcctcggcggtgtttgcgtggggagttggcgaatgagccgagatgtccgagttgcgatact tctgtgttgtataactggattgagaagacgagtaagtttattaagggggttggatgcggatcgtatggtttgtattggt gatgagggcttcggtctcaacatcgactcggacggcagctacccttatcaattctccgagggcttgaactttacgatg aacctcggtatcgatactattgactttggtaccctccacttgtaccctgatagctggggcacctccgacgactggggc aacggctggatcaccgcccacggcgcagcctgcaaagcggccggcaagccatgtctcctggaggaatacggagtcacc tcgaaccactgcagtgtggagggctcgtggcagaagacagcgctcagcacaacgggcgtcggcgcggatctgttctgg cagtatggtgatgatttgagtaccgggaagtcgccggatgatgggaatacTatctactatgggactagtgattatcag tgcctggtgacggatcatgttgctgctattgatagcgcctaa</INSDSeq_sequence></INSDSeq></Seque nceData></ST26SequenceListing>

## Claims

1. A mannanase mutant, wherein the mannanase mutant comprises an amino acid sequence having at least 90% identity with SEQ ID NO: 1, and compared with SEQ ID NO: 1, the mannanase mutant comprises at least one amino acid substitution at a position selected from the group consisting of: 49, 58, 90, 99, 120, 124, 130, 132, 136, 140, 148, 154, 206, 212, 257, 263, 268, 273, 296, 297, 302, 306, 315, 339, 353, 360 and 362 of SEQ ID NO: 1.

2. The mannanase mutant of claim 1, wherein the mannanase mutant comprises an amino acid sequence having at least 91%, 92%, 93%, 94%, 95%, 96%, 97%, 98%, or at least 99% identity with SEQ ID NO: 1.

3. The mannanase mutant of claim 1, wherein the mannanase mutant comprises an amino acid sequence having at least 99.1%, 99.2%, 99.3%, 99.4%, 99.5%, 99.6%, 99.7%, 99.8%, or at least 99.9% identity with SEQ ID NO: 1.

4. The mannanase mutant of any one of claims 1-3, wherein the mannanase mutant comprises at least one amino acid substitution selected from the group consisting of D49C/I/N, G58D/E/S, H90L, T99Y, K120Y, N124P, T130D/N/P/Q/R/S, Y132F, S136M, S140L/K, T148K, D154P, F206Y, A212N/S/P, L257F, S263R, D268A/E/G/P, G273P, V296I, T297Y, S302M/E, S306M/Q, T315P, N339F, T353L/F, D360E/N/K and A362R.

5. The mannanase mutant of claim 4, wherein the mannanase mutant comprises an amino acid substitution or a combination thereof selected from the group consisting of: D49C, D49I, D49N, G58D, G58E, G58S, H90L, T99Y, K120Y, N124P, T130D, T130N, T130P, T130Q, T130R, T130S, Y132F, S136M, S140L, S140K, T148K, D154P, F206Y, A212N, A212S, A212P, L257F, S263R, D268A, D268E, D268G, D268P, G273P, V296I, T297Y, S302M, S302E, S306M, S306Q, T315P, N339F, T353L, T353F, D360E, D360N, D360K, A362R,

D49N/G58D, D49N/H90L, D49N/T99Y, D49N/K120Y, D49N/N124P, D49N/T130R, D49N/T130S, D49N/T130P, D49N/Y132F, D49N/S136M, D49N/S140L, D49N/S140K, D49N/T148K, D49N/D154P, D49N/F206Y, D49N/A212N, D49N/A212S, D49N/A212P, D49C/F206Y, D49I/ F206Y, D49N/L257F, D49N/D268A, D49N/D268E, D49N/D268G, D49N/D268P, D49N/G273P, D49N/S302M, D49N/S302E, D49N/S306M, D49N/S306Q, D49NIT315P, D49N/T353L, D49N/D360E, D49C/D360E, D49I/D360E, D49I/D360E, D49N/D360N, D49N/D360K, D49N/A362R, G58D/H90L, G58D/K120Y, G58D/Y132F, G58D/T99Y, G58D/N124P, G58D/T130P, G58D/S136M, G58D/S140K, G58D/T148K, G58D/D154P, G58D/F206Y, G58D/L257F, G58D/D268P, G58D/G273P, G58D/S302M, G58D/S302E, G58D/S306M, G58D/S306Q, G58D/T315P, G58D/T353L, G58D/D360E, H90L/A362R, H90L/G273P, H90L/S302M, H90L/S302E, H90L/S306M, H90L/S306Q, T99Y/N124P, T99Y/Γ130P, T99Y/S136M, T99Y/S140K, T99Y/Γ148K, T99Y/D154P, T99Y/F206Y, T99Y/L257F, T99Y/D268P, T99Y/G273P, T99Y/S302M, T99Y/S302E, T99Y/S306M, T99Y/S306Q, T99Y/T315P, T99Y/T353L, T99Y/D360E, K120Y/N124P, K120Y/T130P, K120Y/S136M, K120Y/S140K, K120Y/L257F, K120Y/D268P, K120Y/T315P, K120Y/T353L, K120Y/D360E, K120Y/A362R, N124P/T130P, N124P/S136M, N124P/S140K, N124P/L257F, N124P/D268P, N124P/T315P, N124P/T353L, N124P/D360E, T130P/S136M, T130P/S140K, T130P/L257F, T130P/D268P, T130P/T315P, T130P/T353L, T130P/D360E, S136M/S140K, S136M/L257F, S136M/D268P, S136M/T315P, S136M/T353L, S136M/D360E, S140K/L257F, S140K/D268P, S140K/T315P, S140K/T353L, S140K/D360E, T148K/D154P, T148K/F206Y, T148K/L257F, T148K/D268P, T148K/G273P, T148K/S302M, T148K/S302E, T148K/S306M, T148K/S306Q, T148K/T315P, T148K/T353L, T148K/D360E, D154P/L257F, D154P/D268P, D154P/G273P, D154P/S302M, D154P/S302E, D154P/S306M, D154P/S306Q, D154P/T315P, D154P/T353L, D154P/D360E, F206Y/G273P, F206Y/S302M, F206Y/S302E, F206Y/S306M, F206Y/S306Q, F206Y/T315P, F206Y/T353L, F206YID360E, A212N/D268P, A212N/T315P, A212N/T353L, A212N/D360E, L257F/D268P, L257F/T315P, L257F/T353L, L257F/D360E, D268P/T315P, D268P/T353L, D268P/D360E, G273P/S302M, G273P/S302E, G273P/S306M, G273P/S306Q, G273P/T315P, G273P/T353L, G273P/D360E, G273P/A362R, S302M/S306M, S302M/S306Q, S302M/T315P, S302M/T353L, S302M/D360E, S302M/A362R, S302E/S306M, S302E/S306Q, S302E/T315P, S302E/T353L, S302E/D360E, S302E/A362R, S306M/T315P, S306M/T353L, S306M/D360E, S306M/A362R, S306Q/T315P, S306Q/T353L, S306Q/D360E, S306Q/A362R, T315P/T353L, T315P/D360E, T353L/D360E, T315P/A362R, T353L/A362R, D360E/A362R,
D49N/G58D/N124P, D49N/G58D/T130P, D49N/T99Y/N124P, D49N/T99Y/S136M, D268P/T315P/D360E, T130P/ L257F/D268P, N124P/T130P/D268P, N124P/T130P/ D360E, N124P/S302M/S306M, D49N/G58D/L257F, D49N/G58D/D268P, D49N/G58D/A362R, D49N/T99Y/S140K, D49N/T99Y/L257F, D49N/T99Y/D268P, D49N/G58D/T315P, D49N/G58D/D360E, G58D/N124P/T130P, G58D/N124P/L257F, G58D/N124P/ D268P, G58D/ D268P/T315P, L257F/D268P/T315P, N124P/T130P/ D360K, D49N/T99Y/T315P, D49N/T99Y/T353L, D49N/T99Y/D360E, K120Y/N124P/S140K, K120Y/T130P /L257F, K120Y/S136M/D268P, K120Y/S140K/T353L, N124P/T130P/L257F, N124P/T130P/D268P, A212P/L257F/D268P,
D49N/G58D/N124P/T130P, D49N/G58S/ T130P/D268P, G58S/ N124P/T130P/D268P, G58D/N124P/T130P/D268P, N124P/T130P/L257F/D268P, N124P/T130P/S136M/T353L, N124P/T130P/S136M/D360E, S136M/S140K/T315P/T353L, S136M/L257F/T315P/T353L, S136M/D268P/S306M/T315P, S136M/T315P/T353L/D360E, G58D/T99Y/S140K/T315P/T353L, G58D/N124P/T315P/T353L/D360E, G58S/T99Y/N124P/T130P/D268P, G58D/T99Y/N124P/T130P/D268P, G58D/T130P/T315P/T353L/D360E, G58D/K120Y/N124P/S140K/S136M, D49N/T99Y /S136M/G273P/S302M, T99Y/N124P/T130P/L257F/D268P, D49N/G58D/T99Y/N124P/T130P, D49N/G58D/N124P/T130P/D268P, D49N/G58D/N124P/T130P/T315P, Y132F/L257F/S263R/D268P/T315P, Y132F/L257F/S263R/D268P/T315P, Y132F/L257F/S263R/S306M/T315P, Y132F/G273P/S263R/S306M/T315P, Y132F/G273P/S263R/S306M/D360N, D49N/G58D/N124P/T130P/T353L, D49N/G58D/N124P/T130P/D360E, D49N/G58S/N124P/T130P/S136M, A212P/L257F/S263R/D268P/F315P, A212P/L257F/S263R/D268P/T315P, A212P/L257F/S263R/S306M/T315P, A212P/G273P/S263R/S306M/T315P, A212P/G273P/S263R/S306M/D360N, D49NIT99Y IS 140KIT353LID360E, D49N/T99Y/L257F/S302M/A362R, D49N/G58D/N124P/T130P/S140K, D49N/G58D/N124P/T130P/L257F, G5 8D/N124 P/T13 0P/L25 7F/D26 8P, D49N/G58D/N124P/T130P/L257F/D268P, G58D/T99Y/N124P/T130P/L257F/D268P, G58D/N124P/T130P/S136M/L257F/D268P, G58D/N124P/T130P/S140K/L257F/D268P, D154PAL257F/D268P/T315P/T353L/D360E, D154P/D268P/S306Q/T315P/T353L/D360E, D154P/F206Y/D268P/S306Q/T353L/D360E, D49N/N124P/T130P/L257F/T315P/D360E, D49N/ N124P/T130P/L257F/D268P/D360E, T148K/D154P/D268P/S306Q/T315P/T353L, T148K/D154P/D268P/S306M/T315P/T353L, D49N/ N124P/T130P/ L257F/D268P/T315P, D49N/ N124P/F206Y/L257F/D268P/D360K, D49N/ N124P/F206Y/L257F/D268P/T315P, N124P/T130P/L257F/D268P/T315P/D360E, G58D/N124P/T130P/L257F/D268P/T315P, G58D/N124P/T130P/L257F/D268P/T353L, G58D/N124P/T130P/L257F/D268P/D360E, G58D/N124P/T130P/L257F/D268P/T315P, T148K/D154P/ A212P /S306Q/T315P/T353L, D49N/ T130P/ L257F/D268P/T315P/D360E, D49N/ N124P/ L257F/D268P/T315P/D360E, T148K/D154P/ S263R/S306M/T315P/T353L, T148K/D154P/ G273P/S306Q/T315P/T353L, D49N/ N124P/T130P/ D268P/T315P/D360E,

EP 4 403 630 A1

G58D/N124P/T130P/L257F/D268P/G273P/D360E,
D49N/G58D/T99Y/N124P/T130P/L257F/D268P,
D49N/G58D/N124P/T130P/S136M/L257F/D268P,
D49N/G58D/N124P/T130P/S140K/L257F/D268P,
D49N/G58D/N124P/T130P/L257F/D268P/T315P,
D49N/G58D/N124P/F130P/L257F/D268P/T353L,
D49N/G58D/N124P/T130P/D268P/T315P/D360E,
D49N/G58D/N124P/T130P/ L257F/ T315P/D360E,
H90L/N124P T130P/L257F/D268P/G273P/D360E,
H90L/N124P/T13 0P/L2 5 7F/D26 8P/G273P/A3 62R,
D49N/G58D/NI24P/TI30P/L257F/D268P/D360K,
G58D/N124P/T130P/L257F/D268P/T315P/D360E,
D49N/ N124P/T130P/ L257F/D268P/T315P/D360E,
D49N/G58D/ T130P/ L257F/D268P/T315P/D360E,
D49N/G58D/N124P/ L257F/D268P/T315P/D360E,
D49N/G58D/N124P/T130P/ L257F/D268P/ D360E,
G58D/T99Y/N124P/T130P/L257F/D268P/S302M/S306M,
G58D/N124P/T130P/S136M/L257F/D268P/S302M/T315P,
G58D/N124P/T130P/S140K/L25F/D268P/S302M/D360E,
D49N/G58D/N124P/T130P/L257F/D268P/T315P/T353L,
D49N/G58D/N124P/T130P/S140K/L257F/D268P/T315P,
D49N/G58D/N124P/T130P/S136M/L257F/D268P/T315P,
D49N/G58D/T99Y/N124P/T130P/L257F/D268P/T315P,
T148K/S140K/D154P/L257F/D268P/T315P/T353L/D360E,
D49N/G58D/N124P/T130P/L257F/D268P/T315P/D360E,
D 154P/D268P/S3 02M/S3 06M/T31 SP/T3 53 L/D3 60E/A3 62R,
D 154P/D268P/S3 02M/S3 06M/T31 SP/T3 53 L/D3 60E/A3 62R,
D49N/G58D/N124P/T130P/S140K/L257F/D268P/T353L,
D49N/G58D/N124P/T130P/S136M/L257F/D268P/T353L,
D49N/G58D/H90L/T130P/S136M/L257F/S306M/T353L,
D49N/G58D/H90L/T130P/S136M/G273P/S306M/T353L,
D49N/G58D/N124P/F130P/S136M/S140K/L257F/D268P,
D49N/G58D/N124P/T130P/L257F/245P/G273P/A362R,
H90L/T99Y/N124P/T130P/L257F/D268P/S302M/S306M,
H90L/N124P/T130P/S136M/L257F/D268P/S302M/T315P,
H90L/N124P/T130P/S140K/L257F/D268P/S302M/D360E,
T99Y/N124P/T130P/Y132F/D268P/S302P/S306M/T315P,
D49N/G58D/N124P/T130P/L257F/D268P/T315P/D360E/T353L,
D49N/G58D/N124P/T130P/S136M/L257F/D268P/T315P/D360E,
D49N/G58D/N124P/T130P/S140K/L257F/D268P/T315P/D360E,
D49N/G58D/N124P/T130P/L257F/D268P/T315P/T353L/D360E,
D49N/G58D/T99Y/N124P/T130P/L257F/D268P/T315P/D360E,
T99Y/N124P/T130P/L257F/D268P/S302M/T315P/D360E/A362R,
K120Y/N124P/T130P/S136M/L257F/D268P/T315P/T3 53L/D360E,
D49N/G58D/N124P/T130P/S136M/S140K/L257F/D268P/T315P/T3 53L/D360E,
D49N/G58D/N124P/T130P/S136M/L257F/D268P/V296I/T297Y/T315P/D360E,
D154P/L257F/D268P/S302M/S306M/T315P/T353L/D360E/A362R,
D49N/G58D/N124P/T130P/S136M/S140K/L257F/D268P/T315P/D360E,
D154P/L257F/D268P/S302M/S306M/T315P/T353L/D360E/A362R,
D49N/G58D/N124P/T130P/S136M/L257F/D268P/T31SP/T353L/D360E,
D49N/G58D/N124P/T130P/S140K/L257F/D268P/T315P/T353L/D360E,
D49N/G58D/N124P/F130P/S136M/L257F/D268P/V296I/F315P/D360E,
D49N/G58D/N124P/T130P/S136M/L257F/D268P/T297Y/T315P/D360E,
D49N/G58D/N124P/T130P/L257F/D268P/G273P/S306M/T315P/T353L/D360E,
D49N/G58D/T99Y/N124P/T130P/L257F/D268P/S302M/T315P/D360E/A362R,
G58D/T99Y/N124P/T130P/S136M/S140K/D154P/L257F/D268P/T315P/D360E/A362R,
T130P/Y132F/S136M/S140K/D268P/G273P/S302M/S306M/T315P/T353L/D360E/A362R,
D49N/G58D/T99Y/N124P/T130P/S136M/S140K/L257F/D268P/T315P/T353L/D360K,

16

D49N/G58D/H90L/K120Y/N124P/T130P/S136M/L257F/D268P/T31SP/T353L/D360E,
D49N/G58D/T99Y/N124P/T130P/S136M/S140K/L257F/D268P/T315P/T353L/D360E,
D49N/G58D/N124P/T130P/T148K/S140K/D154P/L257F/D268P/T315P/T353L/D360E,
D49N/G58D/N124P/T130P/S136M/L257F/D268P/V296I/T297Y/T315P/N339F/D360E,
D49N/G58D/T99Y/N124P/T1130P/Y132F/D154P/D268P/G273P/S302P/S306M/T315P,
T99Y/N124P/T130P/Y132F/S136M/S140K/D268P/S302M/S306M/T315P/T353L/D360E/ A362R,
H90L/N124P/T130P/Y132F/S136M/S140K/D268P/S302M/S306M/T315P/T353L/D360E/ A362R,
H90L/K120Y/T130P/Y132F/S136M/S140K/D268P/S302M/S306M/T315P/T353L/D360K/ A362R,
N124P/T130P/Y132F/S136M/S140K/L257F/D268P/S302M/S306M/T315P/T353L/D360E/ A362R,
T99Y/S113M/S117K/N124P/T130P/Y132F/D268P/S302P/S306M/T315P/T330L/D337E/A 339R,
T99Y/N124P/T130P/Y132F/S136M/S140K/L257F/D268P/S302M/S306M/T315P/T353L/D 360E/A362R,
T148K/D154P/F206Y/A212P/L257F/S263R/D268P/G273P/S302M/S306M/T315P/T353F/ D360E/A362R,
T99Y/T148K/D154P/F206Y/A212P/L257F/S263R/D268P/G273P/S302M/S306M/T315P/T 353F/D360E/A362R,
T99Y//N124P/T148K/D154P/F206Y/A212P/L257F/S263R/D268P/G273P/S302M/S306M/ T315P/T353F/D360E/A362R,
D49N/G58D/N124P/T130P/T148K/A212P/L257F/S263R/D268P/G273P/S302M/S306M/T 315P/T353F/D360E/A362R,
D49N/G58D/T99Y/T130P/T148K/A212P/L257F/S263R/D268P/G273P/S302M/S306M/T3 15P/T353F/D360E/A362R,
D49N/G58D/T99Y/T130P/T148K/A212P/L257F/S263R/D268P/G273P/S302M/S306M/T3 15P/T353F/D360E/A362R,
D49N/G58D/T99Y/T130P/T148K/D154P/A212P/L257F/S263R/D268P/G273P/S302M/S30 6M/T315P/T353F/D360E/A362R,
D49N/G58D/T99Y/T130P/T148K/D154P/A212P/L257F/S263R/D268P/G273P/S302M/S30 6M/T315P/T353F/D360E/A362R,
G58D/T99Y/N124P/T130P/D154P/F206Y/A212P/L257F/S263R/D268P/G273P/S302M/S3 06M/T315P/T353F/D360E/A362R,
G58D/H90L/T99Y/N124P/T130P/D154P/F206Y/A212P/L257F/S263R/D268P/G273P/S30 2M/S306M/T315P/T353F/D360K/A362R,
G58D/H90L/T99Y/N124P/T130P/D154P/F206Y/A212P/L257F/S263R/D268P/G273P/S30 2E/S306M/T315P/T353F/D360E/A362R,
G58D/H90L/T99Y/N124P/T130P/D154P/F206Y/A212P/L257F/S263R/D268P/G273P/S30 2E/S306M/T315P/T353F/D360N/A362R, and
G58D/H90L/T99Y/N124P/T130P/D154P/F206Y/A212P/L257F/S263R/D268P/G273P/S30 2E/S306M/T315P/T353L/D360K/A362R.

6. A DNA molecule encoding the mannanase mutant of any one of claims 1-5.

7. A recombinant expression plasmid comprising the DNA molecule of claim 6.

8. A host cell comprising the recombinant expression plasmid of claim 7.

9. The host cell of claim 7, wherein the host cell is selected from the group consisting of *Pichia pastoris* and *Trichoderma reesei*.

10. Use of the mannanase mutant of any one of claims 1-5 in the field of feeds.

<div align="center">**INTERNATIONAL SEARCH REPORT**</div>

| International application No. |
| --- |
| **PCT/CN2022/119328** |

**A. CLASSIFICATION OF SUBJECT MATTER**

C12N 9/42(2006.01)i; C12N 15/56(2006.01)i; C12N 15/81(2006.01)i; C12N 1/19(2006.01)i; C12R 1/84(2006.01)i

According to International Patent Classification (IPC) or to both national classification and IPC

**B. FIELDS SEARCHED**

Minimum documentation searched (classification system followed by classification symbols)

C12N; C12R

Documentation searched other than minimum documentation to the extent that such documents are included in the fields searched

Electronic data base consulted during the international search (name of data base and, where practicable, search terms used)

WPABS, CNTXT, ENTXTC, DWPI, VEN, CNKI, 万方, WANFANG, ISI WEB OF SCIENCES, PUBMED, GenBank, STN: 申请人, 发明人, 耐高温, 耐热, 甘露聚糖酶, 突变, heat resistance, mannanase , mutant, D49C, SEQ ID NO: 1

**C. DOCUMENTS CONSIDERED TO BE RELEVANT**

| Category* | Citation of document, with indication, where appropriate, of the relevant passages | Relevant to claim No. |
| --- | --- | --- |
| A | CN 103525790 A (QINGDAO GENYUAN BIOLOGICAL TECHNOLOGY GROUP CO., LTD.) 22 January 2014 (2014-01-22)<br>entire document | 1-10 (in part) |
| A | CN 110577946 A (QINGDAO RED CHERRY BIOTECHNOLOGY CO., LTD.) 17 December 2019 (2019-12-17)<br>entire document | 1-10 (in part) |
| A | CN 111363735 A (OCEAN UNIVERSITY OF CHINA) 03 July 2020 (2020-07-03)<br>entire document | 1-10 (in part) |
| A | CN 104726481 A (OIL CROPS RESEARCH INSTITUTE, CHINESE ACADEMY OF AGRICULTURAL SCIENCES) 24 June 2015 (2015-06-24)<br>entire document | 1-10 (in part) |
| A | CN 103255119 A (CENTRAL SOUTH UNIVERSITY) 21 August 2013 (2013-08-21)<br>entire document | 1-10 (in part) |
| A | CN 110669748 A (TIANJIN INSTITUTE OF INDUSTRIAL BIOTECHNOLOGY, CHINESE ACADEMY OF SCIENCES) 10 January 2020 (2020-01-10)<br>entire document | 1-10 (in part) |

☑ Further documents are listed in the continuation of Box C.     ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **30 November 2022** | **13 December 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/119328** |

### C. DOCUMENTS CONSIDERED TO BE RELEVANT

| Category* | Citation of document, with indication, where appropriate, of the relevant passages | Relevant to claim No. |
| --- | --- | --- |
| A | CN 111117987 A (QINGDAO VLAND BIOTECH GROUP CO., LTD.) 08 May 2020 (2020-05-08)<br>    entire document | 1-10 (in part) |
| A | WO 2021152123 A1 (NOVOZYMES A/S) 05 August 2021 (2021-08-05)<br>    entire document | 1-10 (in part) |
| A | WO 2019214702 A1 (FEED RESEARCH INSTITUTE, CHINESE ACADEMY OF AGRICULTURAL SCIENCES) 14 November 2019 (2019-11-14)<br>    entire document | 1-10 (in part) |
| A | 罗长财等 (LUO, Changcai et al.). "一株产耐高温β-甘露聚糖酶菌株的筛选鉴定 (Screening and Identification of a Strain Highly Producing Thermostable β-Mannanase)"<br>*生物技术通讯 (Letters in Biotechnology)*, Vol. 29, No. 02, 30 March 2018 (2018-03-30),<br>    pp. 233-237 | 1-10 (in part) |
| A | 周海燕等 (ZHOU, Haiyan et al.). "点突变对枯草芽孢杆菌甘露聚糖酶结构和功能的影响 (Non-official translation: Effect of Point Mutation on Structure and Function of Bacillus Subtilis Mannanase Structure)"<br>*高等函授学报(自然科学版) (Journal of Higher Correspondence Education (Natural Sciences))*, Vol. 22, No. 01, 26 February 2008 (2008-02-26),<br>    pp. 9-12 | 1-10 (in part) |

Form PCT/ISA/210 (second sheet) (January 2015)

**INTERNATIONAL SEARCH REPORT**

| International application No. |
| --- |
| **PCT/CN2022/119328** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.    ☑   forming part of the international application as filed:

           ☑   in the form of an Annex C/ST.25 text file.

           ☐   on paper or in the form of an image file.

     b.    ☐   furnished together with the international application under PCT Rule 13*ter*.1(a) for the purposes of international search only in the form of an Annex C/ST.25 text file.

     c.    ☐   furnished subsequent to the international filing date for the purposes of international search only:

           ☐   in the form of an Annex C/ST.25 text file (Rule 13*ter*.1(a)).

           ☐   on paper or in the form of an image file (Rule 13*ter*.1(b) and Administrative Instructions, Section 713).

2.   ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

     [1]   The sequence table is submitted in the form of a C/ST.26 file.

Form PCT/ISA/210 (continuation of first sheet) (January 2015)

**INTERNATIONAL SEARCH REPORT**

| International application No. |
|---|
| **PCT/CN2022/119328** |

| Box No. III      Observations where unity of invention is lacking (Continuation of item 3 of first sheet) |
|---|

This International Searching Authority found multiple inventions in this international application, as follows:

[1]    Claim 1 comprises a mannanase mutant relating to 27 different mutation sites and a combination thereof. Claim 5 further defines 374 variants obtained by combining two or more of the 27 sites. Hence, the present application relates to 27 single mutation variants and 374 multi-mutation combined variants in total, that is, claims 1-10 comprise 401 inventions relating to mannanase mutants having different site mutations, and related products or applications thereof.

[2]    The mutation sites of the 401 variants are different; the consensus sequence SEQ ID NO: 1 other than the mutation sites is disclosed in D1 (CN111117987A, 08 May 2020); D1 discloses a high-specific-activity acidic mannanase mutant having at least 95% homology to the amino acid sequence SEQ ID NO: 1 of mannanase, the SEQ ID NO: 1 being exactly the same as the SEQ ID NO: 1 of the present application. Hence, the consensus sequence of the 401 groups of variants is not a special technical feature. Therefore, the 401 inventions comprised in claims 1-10 and relating to different mutation sites and a combination thereof do not have a special technical feature that makes a contribution over the prior art as defined in PCT Rule 13.2. Therefore, the present application does not comply with unity defined in PCT Rule 13.1.

1. ☐    As all required additional search fees were timely paid by the applicant, this international search report covers all searchable claims.

2. ☐    As all searchable claims could be searched without effort justifying additional fees, this Authority did not invite payment of additional fees.

3. ☐    As only some of the required additional search fees were timely paid by the applicant, this international search report covers only those claims for which fees were paid, specifically claims Nos.:

4. ☑    No required additional search fees were timely paid by the applicant. Consequently, this international search report is restricted to the invention first mentioned in the claims; it is covered by claims Nos.: **1-10 (in part)**

**Remark on Protest**    ☐    The additional search fees were accompanied by the applicant's protest and, where applicable, the payment of a protest fee.

                             ☐    The additional search fees were accompanied by the applicant's protest but the applicable protest fee was not paid within the time limit specified in the invitation.

                             ☐    No protest accompanied the payment of additional search fees.

Form PCT/ISA/210 (continuation of first sheet) (January 2015)

**INTERNATIONAL SEARCH REPORT**
Information on patent family members

International application No.

**PCT/CN2022/119328**

| Patent document cited in search report | | | Publication date (day/month/year) | Patent family member(s) | | | Publication date (day/month/year) |
|---|---|---|---|---|---|---|---|
| CN | 103525790 | A | 22 January 2014 | None | | | |
| CN | 110577946 | A | 17 December 2019 | None | | | |
| CN | 111363735 | A | 03 July 2020 | None | | | |
| CN | 104726481 | A | 24 June 2015 | None | | | |
| CN | 103255119 | A | 21 August 2013 | None | | | |
| CN | 110669748 | A | 10 January 2020 | None | | | |
| CN | 111117987 | A | 08 May 2020 | None | | | |
| WO | 2021152123 | A1 | 05 August 2021 | BR | 112022014946 | A2 | 04 October 2022 |
| | | | | CN | 115052981 | A | 13 September 2022 |
| WO | 2019214702 | A1 | 14 November 2019 | EP | 3792356 | A1 | 17 March 2021 |
| | | | | US | 2021189366 | A1 | 24 June 2021 |
| | | | | CN | 108559739 | A | 21 September 2018 |

Form PCT/ISA/210 (patent family annex) (January 2015)

**REFERENCES CITED IN THE DESCRIPTION**

**Patent documents cited in the description**

- CN 202111097561

**Non-patent literature cited in the description**

- MOLECULAR CLONING: A LABORATORY MANU-AL. 2001
- CURRENT PROTOCOLS IN MOLECULAR BIOLO-GY. 2003